# EUROPÄISCHE PATENTSCHRIFT

(11) **EP 0 555 260 B1**
(45) Veröffentlichungstag und Bekanntmachung des Hinweises auf die Patenterteilung: **20.09.1995**
(21) Anmeldenummer: 91918263.4
(22) Anmeldetag: 16.10.1991
(51) Int. Cl.: C07C 1/26, C07C 15/14, C07C 25/18

(54) **VERFAHREN ZUR HERSTELLUNG VON BIARYLEN**
PROCESS FOR THE PRODUCTION OF BIARYLS
PROCEDE DE PRODUCTION DE BIARYLES

(30) Priorität: 26.10.1990 DE 4034109
(43) Veröffentlichungstag der Anmeldung: 18.08.1993
(73) Patentinhaber: HOECHST AKTIENGESELLSCHAFT, 65926 Frankfurt am Main (DE)
(72) Erfinder: SCHACH, Thomas, D-6238 Hofheim am Taunus (DE); PAPENFUHS, Theodor, D-6000 Frankfurt am Main (DE); HACKENBRUCH, Joachim, D-6500 Mainz (DE)
(86) Internationale Anmeldenummer: EP9101968
(87) Internationale Veröffentlichungsnummer: WO9207809

(56) Entgegenhaltungen:
- EP-A- 0 206 543
- EP-A- 0 318 634
- US-A- 4 727 185

## Beschreibung

Die vorliegende Erfindung betrifft ein verbessertes Verfahren zur Herstellung von Biarylen durch Enthalogenierung und Dimerisierung von Arylhalogeniden in Gegenwart eines Palladiumkatalysators auf einem Trägermaterial, eines Reduktionsmittels, eines Halogenwasserstoffakzeptors, eines Polyethers oder Polyethergemisches und von Wasser.

Biaryle haben eine große Bedeutung als Synthesebausteine für pharmazeutische Produkte, werden aber auch im Bereich des Pflanzenschutzes und der Flüssigkristall-Technologie benötigt, wobei fluorierten Biarylen eine besondere Bedeutung zukommt. Die gezielte Einführung eines Fluorsubstituenten in einen Biarylkörper ist in vielen Fällen nur durch eine aufwendige und dadurch teure Balz-Schiemann-Reaktion möglich. Die Grenzen dieser Reaktion liegen zum einen in der Verfügbarkeit der Ausgangsverbindungen und zum anderen in deren teilweise hohen Toxizität (z.B. Benzidin), beides Argumente, die ihr Synthesepotential stark einschränken.

Für die Synthese der gewünschten Biaryle durch Aufbau-Reaktionen aus bereits fluorierten Verbindungen stehen eine Anzahl von Kupplungsreaktionen zur Verfügung. Diese erfordern zum Teil einen erheblichen technischen Aufwand (Elektroreduktive Kupplungen, Grignard-Reaktionen) oder liefern die gewünschten Biaryle nur in mäßigen Ausbeuten (Ullmann-Kupplung) bzw. in schlechten Selektivitäten (Gomberg-Bachmann-Reaktion).

Eine weitere Möglichkeit zur Darstellung von Biarylen ist durch eine Dehalogenierungs-Dimerisierungs-Reaktion in Gegenwart von Edelmetallkontakten, eines Reduktionsmittels und eines Halogenwasserstoffakzeptors gegeben (M. Busch und W. Weber; Journal f. prakt. Chemie, 146, 1-55, 1936; F.R. Mayo und M.D. Hurwitz, J. Chem. Soc., 71, 776-779, 1949; P. Bamfield und P.M. Quan, Synthesis 7, 537-538, 1978). Die Problematik dieses Reaktionstyps liegt in der häufig ungünstigen Selektivität, mit der das gewünschte Biaryl erhalten wird. So fallen neben den Biarylen stets auch die enthalogenierten Ausgangsverbindungen an, die damit die Ausbeuten zum Teil beträchtlich mindern. Ein weiterer Nachteil liegt in der schnellen Selektivitätsabnahme des Katalysators bei mehrfachem Wiedereinsatz, wodurch der technischen Anwendung dieser Reaktion starke Grenzen gesetzt werden (s. z.B.: F.R. Mayo and M.D. Hurwitz, J. Chem. Soc. 71, 776-779, 1949).

Mit verschiedenen Reduktionsmitteln wurde diese Synthesemethode zur Darstellung von Biarylen benutzt (EP 206 543), insbesondere zur Synthese von 3,3',4,4'-Biphenyltetracarbonsäure (UP 4 727 185, EP 318 634). Die bislang bekannten Reaktionen dieses Typs liefern zumeist nur mäßige Ausbeuten der gewünschten Biaryle.

Es wurde nun überraschenderweise gefunden, daß man Biaryle der allgemeinen Formel (1)

R¹ₘ - Ar - Ar - R¹ₘ (1)

in welcher Ar einen Phenylen- oder Naphthylenrest, R₁ ein Fluor- oder Chloratom oder einen unverzweigten oder verzweigten Alkyl(C₁-C₆)-, Alkyl(C₁-C₆)-O-, Alkyl(C₁-C₆)-CO- oder Alkyl(C₁-C₆)-SO₂-Rest und m die Zahl der Reste R¹ der am Rest Ar substituierten 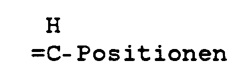
bedeuten, in vorteilhafter Weise in guten Ausbeuten und hoher Selektivität herstellen kann, indem man eine Verbindung der allgemeinen Formel (2)

R¹ₘ - Ar - X (2)

in welcher Ar, R¹ und m die vorstehend genannten Bedeutungen haben und X ein Chlor- oder Bromatom darstellt, in Gegenwart eines Palladium-Katalysators auf einem Trägermaterial, eines Reduktionsmittels, eines Halogenwasserstoffakzeptors, eines Polyethers oder Polyethergemisches und von Wasser bei Temperaturen von 50 bis 120°C, vorzugsweise von 70 bis 110°C, dehalogeniert und dimerisiert.

Es wurde insbesondere gefunden, daß die dehalogenierende Dimerisierung in Gegenwart eines Polyethers oder Polyethergemisches einen entscheidenden Einfluß auf die Selektivität der Kupplungsreaktion hat mit der Folge einer wesentlichen Ausbeutesteigerung.

Als Polyether oder Polyethergemische kommen beispielsweise solche der allgemeinen Formel (3)

R³ - (O - CH₂ - CH₂ -)ₚ OR⁴ (3)

in Frage, in welcher R³, R⁴ gleiche oder verschiedene, lineare oder verzweigte Alkyl(C₁-C₆)-Reste, wie beispielsweise Methyl-, Ethyl-, Propyl-, Butyl-, Pentyl, Hexyl, i-Propyl- oder i-Butylgruppen bedeuten, und p für eine Zahl von 1 bis 20 steht.

Der Polyether oder das Polyethergemisch wird in Mengen von 0,1 bis 500 Gew.-%, vorzugsweise von 1 bis 100 Gew.-%, bezogen auf eingesetztes Arylhalogenid, angewandt.

Durch die Anwesenheit von Polyethern oder Polyethergemischen bei der enthalogenierenden Dimerisierung lassen sich neben einem deutlichen Selektivitätszuwachs eine Erhöhung der Katalysatoraktivität feststellen mit der Folge, daß insbesondere bei der Rückführung von beim erfindungsgemäßen Verfahren bereits gebrauchtem Katalysator kaum Selektivitätsverluste festzustellen sind, was in deutlichem Gegensatz zur lösungsmittelfreien Reaktionsvariante steht.

Als Halogenwasserstoffakzeptor dient zweckmäßigerweise eine anorganische Alkalimetall- oder Erdalkalimetallverbindung, wie beispielsweise Lithium-, Natrium-, Kalium-, Rubidium-, Caesium-, Magnesium-, Calcium-, Barium- oder Strontiumhydroxid, -carbonat oder -hydrogencarbonat oder Mischungen daraus. Bevorzugt werden die Natriumverbindungen, insbesondere Natriumhydroxid, verwendet. Es kann aber beispielsweise auch Natriummethylat als Akzeptor eingesetzt werden.

Es ist zweckmäßig, den Halogenwasserstoffakzeptor in Form einer wäßrigen Lösung, enthaltend 50 bis 500 mol%, vorzugsweise 100 bis 350 mol%, besonders bevorzugt 150 bis 200 mol%, bezogen auf 1 mol eingesetztes Arylhalogenid, anzuwenden. Die Konzentration der wäßrigen Alkalimetall- oder Erdalkalimetallverbindung übt einen entscheidenden Einfluß auf die Selektivität der Kupplungsreaktion aus, wobei es zweckmäßig ist, Konzentrationen von 5 bis 50 Gew.-%, vorzugsweise von 15 bis 40 Gew.-%, anzuwenden.

Der erfindungsgemäß in Frage kommende Palladiumkatalysator wird in Form von metallischem Palladium auf einem Trägermaterial eingesetzt. Als Trägermaterial kommen beispielsweise Aktivkohle, Kaliumcarbonat, Bariumcarbonat, Silizium, Aluminium,Titanoxid oder Magnesium in Frage. Palladium auf Aktivkohle hat sich dabei als günstigste Katalysatorform erwiesen.

Der Gehalt an metallischem Palladium liegt im Bereich von 0,1 - 20 Gew.-%, bezogen auf das Trägermaterial; bevorzugt werden Katalysatoren mit 1 - 10 Gew.%, vorzugsweise 5 Gew.%.

Es wurde festgestellt, daß die Menge des eingesetzten Katalysators einen entscheidenden Einfluß auf die Selektivität der Reaktion besitzt. Zu hohe Katalysator-Mengen führen zu unerwünschten Nebenreaktionen, während zu geringe Mengen des Palladium-Katalysators ein vorzeitiges Abbrechen der Reaktion zur Folge hat bzw. sehr lange Reaktionszeiten und ein erhöhter Anteil an ungekuppelten reduzierten Ausgangsaromaten in Kauf genommen werden muß. Im allgemeinen wird der Katalysator in Mengen von 0,001 bis 50 mmol, bevorzugt von 0,5 bis 2 mmol Palladium, bezogen auf ein Mol des eingesetzten Arylhalogenids, eingesetzt.

Zum Wiedereinsatz kann der Katalysator unbehandelt weiterverwendet werden oder mit Alkoholen (z.B. Methanol, Ethanol), Polyethern, Ethern bzw. Wasser oder Wasserdampf vorbehandelt werden.

Als Reduktionsmittel können bei der vorliegenden Erfindung beispielsweise Alkohole, Formaldehyd, Formiate oder Hydrazin verwendet werden. Einzelbeispiele hierfür sind Methanol, Glyzerin, Ethylenglykol, Formalin, Paraformaldehyd und Natriumformiat. Bevorzugt verwendet werden mehrwertige Alkohole, wie beispielsweise Ethylenglykol oder Glyzerin. Das Reduktionsmittel wird in der Regel in einer Menge von 0,1 bis 20 mol, bevorzugt von 0,1 bis 1 mol pro mol eingesetztem Arylhalogenid verwendet.

Im allgemeinen handelt es sich beim Reaktionsmedium um ein dreiphasiges System aus organischer und wäßriger Phase sowie dem heterogenen Palladium-Katalysator, weshalb eine gute Rührung von großer Bedeutung ist.

Zu den weiter oben angegebenen Temperaturbereichen des erfindungsgemäßen Verfahrens sei noch angemerkt, daß bei Temperaturen über 120°C ein Arbeiten unter Druck erforderlich ist. Beim Arbeiten unter 50°C wird die Reaktion sehr langsam; außerdem läuft sie nicht mehr vollständig ab.

Im bevorzugten Temperaturbereich liegen die Reaktionszeiten zwischen 0,5 und 100 Stunden, abhängig vom eingesetzten Arylhalogenid, der Katalysatorkonzentration, der Basenmenge, der Basenkonzentration, dem Anteil an Polyethern und der Menge und Art des verwendeten Reduktionsmittels. Die Reaktion verläuft zu Beginn sehr rasch, sodaß bereits nach 2 Stunden Umsätze von 50 bis 70 mol% erhalten werden. Um Umsätze über 95 mol% zu erreichen, sind zum Teil lange Reaktionszeiten erforderlich, sodaß ein vorzeitiges Abbrechen der Reaktion (80-95 mol% Umsatz) günstig ist.

Die Reaktion der vorliegenden Erfindung kann unter Schutzgas, wie beispielsweise Argon oder Stickstoff, durchgeführt werden. Bevorzugt wird das Arbeiten in Anwesenheit von Luftsauerstoff.

Das eingesetzte Arylhalogenid kann flüssig oder fest sein. Es soll bei Reaktionstemperatur aber vollständig flüssig bzw. vollständig in Lösung sein.

Prinzipiell können auch unterschiedliche Arylhalogenide gleichzeitig für die erfindundungsgemäße Reaktion eingesetzt werden. Dabei kommt es zur Bildung von Gemischen von Biarylen, die aber unter Umständen schwierig zu trennen sind. Das erfindungsgemäße Verfahren kann auch bei anderen isocyclischen Chlor- bzw. Bromaromaten sowie bei heterocyclischen Chlor- bzw. Bromaromaten angewandt werden.

Das hier beschriebene Verfahren zur Darstellung von Biarylen führt zu deutlich höheren Selektivitäten und Ausbeuten als dies bei vergleichbaren bekannten Reaktionen der Fall ist. So wird z.B. in Synthesis 7, 537-538, 1978 (P. Bamfield und P.M. Quan) die Dimerisierung von 2-Bromtoluol beschrieben, die das 2,2'-Bitolyl lediglich in Ausbeuten von 33 % liefert. EP 206 543 beschreibt die analoge Reaktion mit 2-Chlortoluol und mit 2-Bromtoluol mit Ausbeuten von 60 und 55 mol%. Die damit vergleichbare Dimerisierungsreaktion von 2-Brom-5-fluortoluol liefert nach dem hier beschriebenen Verfahren deutlich bessere Ausbeuten. Analoges gilt auch für die Dimerisierung von Chlorbenzol bzw. Brombenzol, deren Ausbeuten von 48 % und 30 - 65 % (Synthesis 7) sich mit dem hier beschriebenen Verfahren und den entsprechenden fluorierten Ausgangsverbindungen deutlich übertreffen lassen.

Die nachstehenden Beispiele dienen zur näheren Erläuterung des erfindungsgemäßen Verfahrens.

### Beispiel 1

In einem 1 Liter Dreihalskolben mit Rührer, Innenthermometer und Rückflußkühler werden 457,1 g Natronlauge 35%ig, 350,0 g 4-Bromfluorbenzol (BrFB), 175,0 g Diethylenglykoldimethylether, 20,0 g Polyethylenglykoldimethylether 500 und 5,8 g Pd/C (5 %ig, 50 % wasserfeucht) vorgelegt. Die Reaktionssuspension wird auf 100°C erhitzt und innerhalb von 2 Stunden mit 62,1 g Ethylenglykol versetzt. Für weitere 16 Stunden verbleibt die Reaktionssuspension bei dieser Temperatur. Anschließend wird der Katalysator entfernt und die organische Phase fraktioniert destilliert. GC-Analysen der Reaktionslösung und isolierte Ausbeuten an 4,4'-Difluorbiphenyl siehe nachstehende Tabelle 1.

| | |
|---|---|
| Schmelzbereich: | 89,1 - 91,0°C |
| Erstarrungspunkt: | 88,9°C |

### Beispiel 2

Ansatz und Reaktionsverlauf analog Beispiel 1 mit dem aus Beispiel 1 zurückgeführten Katalysator. GC-Analysen der Reaktionslösung und isolierte Ausbeuten an 4,4'-Difluorbiphenyl siehe nachstehende Tabelle 1.

### Beispiel 3

Ansatz und Reaktionsverlauf analog Beispiel 1 mit dem aus Beispiel 2 zurückgeführten Katalysator. GC-Analysen der Reaktionslösung und isolierte Ausbeuten an 4,4'-Difluorbiphenyl siehe nachstehende Tabelle 1.

### Beispiel 4

In einem 1 Liter Dreihalskolben mit Rührer, Innenthermometer und Rückflußkühler werden 160,0 g NaOH-Prills in 800,0 g H₂O gelöst und zusammen mit 260 g 4-Chlorfluorbenzol (ClFB), 90,0 g Diethylenglykoldimethylether, 20,0 g Polyethylenglykoldimethylether 500 und 8,0 g Pd/C (5%ig, 50 % wasserfeucht) im Reaktionsgefäß vorgelegt. Die Reaktionssuspension wird auf 100°C erhitzt und innerhalb von 4 Stunden mit 84,8 g Glyzerin 87 %ig versetzt. Für weitere 16 Stunden verbleibt die Reaktionssuspension bei dieser Temperatur. Anschließend wird der Katalysator entfernt und die organische Phase fraktioniert destilliert. GC-Analysen der Reaktionslösung und isolierte Ausbeuten an 4,4'-Difluorbiphenyl siehe nachstehende Tabelle 1.

### Beispiel 5

Versuchsdurchführung analog Beispiel 1 mit folgenden Reaktionskomponenten: 114 g Natronlauge 35%ig, 94,5 g 2-Brom-5-fluortoluol (BrFT), 40,0 g Diethylenglykoldimethylether, 5 g Polyethylenglykoldimethylether 500, 2,5 g Pd/C (5%ig, 50 % wasserfeucht) und 21,5 g Glyzerin 87%ig. GC-Analysen der Reaktionslösung und isolierte Ausbeuten an 4,4'-Difluor-2,2'-bitolyl siehe nachstehende Tabelle 1.

| | |
|---|---|
| Erstarrungspunkt: | 22,4°C |

### Beispiel 6

Versuchsdurchführung analog Beispiel 1 mit folgenden Reaktionskomponenten: 114 g Natronlauge 35%ig, 96,5 g 2,4-Difluorbrombenzol (DFBrB), 40,0 g Diethylenglykoldimethylether, 5 g Polyethylenglykoldimethylether 500, 2,0 g Pd/C (5%ig, 50 % wasserfeucht) und 21,5 g Glyzerin 87%ig. Der Katalysator wird abgetrennt, die organische Phase vom Lösungsmittel befreit und das erhaltene Rohprodukt aus Chlorbenzol umkristallisiert. GC-Analysen der Reaktionslösung und isolierte Ausbeuten an 2,2',4,4'-Tetrafluorbiphenyl siehe nachstehende Tabelle 1.

| | |
|---|---|
| Schmelzbereich: | 141,5-145,5°C |
| Erstarrungspunkt: | 138,1°C |

### Vergleichsbeispiel 1

Ansatz und Versuchsdurchführung analog Beispiel 1 ohne Zugabe von Diethylenglykoldimethylether und Polyethylenglykoldimethylether 500. GC-Analysen der Reaktionslösung siehe nachstehende Tabelle 1.

### Vergleichsbeispiel 2

Ansatz und Versuchsdurchführung analog Beispiel 2 ohne Zugabe von Diethylenglykoldimethylether und Polyethylenglykoldimethylether 500. GC-Analysen der Reaktionslösung siehe nachstehende Tabelle 1.

### Vergleichsbeispiel 3

Ansatz und Versuchsdurchführung analog Beispiel 3 ohne Zugabe von Diethylenglykoldimethylether und Polyethylenglykoldimethylether 500. GC-Analysen der Reaktionslösung siehe nachstehende Tabelle 1.

### Vergleichsbeispiel 4

Ansatz und Versuchsdurchführung analog Beispiel 5 ohne Zugabe von Diethylenglykoldimethylether und Polyethylenglykoldimethylether 500. GC-Analysen der Reaktionslösung siehe nachstehende Tabelle 1.

**Tabelle 1**

| Bsp. | Zeit (h) | Temp. (°C) | Ausbeute nach GC mol(%) | | | | isol.Ausb. mol(%) Ar-Ar* | Ar-X |
|---|---|---|---|---|---|---|---|---|
| | | | Umsatz | H-Ar | Ar-Ar | Ar-Ar* | | |
| 1 | 18 | 100 | 100,0 | 10,3 | 89,1 | 89,1 | 87,8 | BrFB |
| 2 | 18 | 100 | 88,7 | 10,8 | 76,4 | 86,3 | 84,8 | BrFB |
| 3 | 20 | 100 | 87,0 | 13,2 | 73,6 | 84,2 | 82,0 | BrFB |
| 4 | 20 | 95 | 87,5 | 20,7 | 66,0 | 75,5 | 73,2 | ClFB |
| 5 | 20 | 100 | 90,7 | 16,6 | 72,8 | 80,4 | 79,5 | BrFT |
| 6 | 18 | 100 | 100 | 11,8 | 87,1 | 87,1 | 76,3 | DFBrB |
| V1 | 18 | 100 | 100,0 | 14,1 | 82,7 | 82,7 | | BrFB |
| V2 | 18 | 100 | 100,0 | 24,4 | 72,8 | 72,8 | | BrFB |
| V3 | 18 | 100 | 98,2 | 27,6 | 68,9 | 70,2 | | BrFB |
| V4 | 20 | 100 | 100,0 | 31,0 | 65,7 | 65,7 | | BrFT |

| | | | | | | | | |
|---|---|---|---|---|---|---|---|---|
| * Ausbeuten bezogen auf umgesetztes Arylhalogenid | | | | | | | | |
| In der letzten Spalte bedeutet B= Benzol und T= Toluol. | | | | | | | | |

## Patentansprüche

1. Verfahren zur Herstellung von Biarylen der allgemeinen Formel (1)
R¹ₘ - Ar - Ar - R¹ₘ (1)
in welcher Ar einen Phenylen- oder Naphthylenrest, R₁ ein Fluor- oder Chloratom oder einen unverzweigten oder verzweigten Alkyl(C₁-C₆)-, Alkyl(C₁-C₆)-O, Alkyl(C₁-C₆)-CO- oder Alkyl(C₁-C₆)-SO₂-Rest und m die Zahl der Reste R¹ der am Rest Ar substituierten 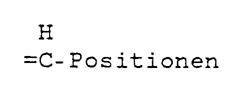 bedeuten, dadurch gekennzeichnet, daß man eine Verbindung der allgemeinen Formel (2)
R¹ₘ - Ar - X (2)
in welcher Ar, R¹ und m die vorstehend genannten Bedeutungen haben und X ein Chlor- oder Bromatom darstellt, in Gegenwart eines Palladium-Katalysators auf einem Trägermaterial, eines Reduktionsmittels, eines Halogenwasserstoffakzeptors, eines Polyethers oder Polyethergemisches und von Wasser bei Temperaturen von 50 bis 120°C dehalogeniert und dimerisiert.

2. Verfahren nach Anspruch 1, dadurch gekennzeichnet, daß im Falle m = 1 R¹ ein Fluoratom ist.

3. Verfahren nach Anspruch 1, dadurch gekennzeichnet, daß im Falle m = 2 R¹ ein Fluoratom ist.

4. Verfahren nach Anspruch 1, dadurch gekennzeichnet, daß im Falle m = 2 am gleichen Rest Ar ein R¹ ein Fluoratom, das andere R¹ eine Methylgruppe ist.

5. Verfahren nach mindestens einem der Ansprüche 1 bis 4, dadurch gekennzeichnet, daß man in Gegenwart eines Polyethers der allgemeinen Formel
R³ - (OCH₂ - CH₂ -)ₚ OR⁴
in welcher R³, R⁴ lineare oder verzweigte Alkyl(C₁-C₆)-Reste und p eine Zahl von 1 bis 20 darstellen, arbeitet.

6. Verfahren nach mindestens einem der Ansprüche 1 bis 5, dadurch gekennzeichnet, daß man in einer Mischung aus Polyethern der in Anspruch 5 genannten allgemeinen Formel arbeitet.

7. Verfahren nach mindestens einem der Ansprüche 1 bis 6, dadurch gekennzeichnet, daß man in Gegenwart von 0,1 bis 500 Gew.-% eines Polyethers oder Polyethergemisches, bezogen auf das eingesetzte Arylhalogenid, arbeitet.

8. Verfahren nach mindestens einem der Ansprüche 1 bis 6, dadurch gekennzeichnet, daß man in Gegenwart von 1 bis 100 Gew.-% eines Polyethers oder Polyethergemisches, bezogen auf das eingesetzte Arylhalogenid, arbeitet.

9. Verfahren nach mindestens einem der Ansprüche 1 bis 8, dadurch gekennzeichnet, daß man in Gegenwart einer anorganischen Alkalimetall- oder Erdalkalimetallverbindung oder von Natriummethylat als Halogenwasserstoffakzeptor arbeitet.

10. Verfahren nach mindestens einem der Ansprüche 1 bis 9, dadurch gekennzeichnet, daß man in Gegenwart von 0,5 bis 10 mol Halogenwasserstoffakzeptor, bezogen auf 1 mol eingesetztes Arylhalogenid, arbeitet.

11. Verfahren nach mindestens einem der Ansprüche 1 bis 10, dadurch gekennzeichnet, daß man in Gegenwart einer 5 bis 50 gewichtsprozentigen wäßrigen Lösung des Halogenwasserstoffakzeptors arbeitet.

12. Verfahren nach mindestens einem der Ansprüche 1 bis 10, dadurch gekennzeichnet, daß man in Gegenwart einer 15 bis 40 gewichtsprozentigen wäßrigen Lösung des Halogenwasserstoffakzeptors arbeitet.

13. Verfahren nach mindestens einem der Ansprüche 1 bis 12, dadurch gekennzeichnet, daß man in Gegenwart eines Palladium-Katalysators auf Aktivkohle oder Calciumcarbonat arbeitet

14. Verfahren nach mindestens einem der Ansprüche 1 bis 13, dadurch gekennzeichnet, daß man in Gegenwart von 0,1 bis 10 Gew.-% Palladium-Katalysator, bezogen auf das Trägermaterial, arbeitet.

15. Verfahren nach mindestens einem der Ansprüche 1 bis 13, dadurch gekennzeichnet, daß man in Gegenwart von 0,001 bis etwa 50 mmol Palladium, bezogen auf 1 mol eingesetztes Arylhalogenid, arbeitet.

16. Verfahren nach mindestens einem der Ansprüche 1 bis 15, dadurch gekennzeichnet, daß man in Gegenwart eines bereits mehrfach bei der Dehalogenierung und Dimerisierung eingesetzten Palladiumkatalysators auf Trägermaterial arbeitet.

17. Verfahren nach mindestens einem der Ansprüche 1 bis 16, dadurch gekennzeichnet, daß man in Gegenwart von einwertigen oder mehrwertigen Alkoholen, Formaldehyd, Formiaten oder Hydrazin als Reduktionsmittel arbeitet.

18. Verfahren nach mindestens einem der Ansprüche 1 bis 16, dadurch gekennzeichnet, daß man in Gegenwart von Methanol, Glyzerin, Ethylenglykol, Formalin, Paraformaldehyd oder Natriumformiat als Reduktionsmittel arbeitet.

19. Verfahren nach mindestens einem der Ansprüche 1 bis 18, dadurch gekennzeichnet, daß man in Gegenwart von 10 bis 200 mol% des Reduktionsmittels, bezogen auf 1 mol eingesetztes Arylhalogenid, arbeitet.

## Claims

1. A process for the preparation of biaryls of the formula (1)
R¹ₘ - Ar - Ar - R¹ₘ (1)
in which Ar is a phenylene or naphthylene radical, R¹ is a fluorine or chlorine atom or an unbranched or branched alkyl(C₁-C₆)-, alkyl(C₁-C₆)-O-, alkyl(C₁-C₆)-CO- or alkyl(C₁-C₆)-SO₂- radical and m is the number of R¹ radicals of the =C(H)- positions substituted on the Ar radical, which comprises dehalogenating and dimerizing a compound of the formula (2)
R¹ₘ - Ar - X (2)
in which Ar, R¹ and m have the meanings cited above and X is a chlorine or bromine atom, in the presence of a palladium catalyst on a support material, of a reducing agent, of a hydrogen halide acceptor, of a polyether or polyether mixture and of water at temperatures from 50 to 120°C.

2. The process as claimed in claim 1, wherein in the case m = 1, R¹ is a fluorine atom.

3. The process as claimed in claim 1, wherein in the case m = 2, R¹ is a fluorine atom.

4. The process as claimed in claim 1, wherein in the case m = 2, on the same Ar radical one R¹ is a fluorine atom and the other R¹ is a methyl group.

5. The process as claimed in at least one of claims 1 to 4, wherein a polyether of the formula
R³ - (OCH₂ - CH₂ - )ₚ OR⁴
in which R³ and R⁴ are linear or branched alkyl(C₁-C₆)-radicals and p is a number from 1 to 20, is used.

6. The process as claimed in at least one of claims 1 to 5, wherein a mixture of polyethers of the formula given in claim 5 is used.

7. The process as claimed in at least one of claims 1 to 6, wherein a polyether or polyether mixture is used at 0.1 to 500 % by weight, related to the aryl halide used.

8. The process as claimed in at least one of claims 1 to 6, wherein a polyether or polyether mixture is used at 1 to 100 % by weight, related to the aryl halide used.

9. The process as claimed in at least one of claims 1 to 8, wherein an inorganic alkali metal or alkaline earth metal compound or sodium methylate is used as hydrogen halide acceptor.

10. The process as claimed in at least one of claims 1 to 9, wherein the hydrogen halide acceptor is used at 0.5 to 10 mol, per mole of aryl halide used.

11. The process as claimed in at least one of claims 1 to 10, wherein a 5 to 50 percent by weight aqueous solution of the hydrogen halide acceptor is used.

12. The process as claimed in at least one of claims 1 to 10, wherein a 15 to 40 percent by weight aqueous solution of the hydrogen halide acceptor is used.

13. The process as claimed in at least one of claims 1 to 12, wherein a palladium catalyst on activated charcoal or calcium carbonate is used.

14. The process as claimed in at least one of claims 1 to 13, wherein a palladium catalyst is used at 0.1 to 10 % by weight, related to the support material.

15. The process as claimed in at least one of claims 1 to 13, wherein palladium is used at 0.001 to 50 mmol, per mole of aryl halide used.

16. The process as claimed in at least one of claims 1 to 15, wherein a palladium catalyst on support material, previously used repeatedly in the dehalogenation and dimerization, is used.

17. The process as claimed in at least one of claims 1 to 16, wherein monohydric or polyhydric alcohols, formaldehyde, formates or hydrazine are used as reducing agent.

18. The process as claimed in at least one of claims 1 to 16, wherein methanol, glycerol, ethylene glycol, formalin, paraformaldehyde or sodium formate are used as reducing agent.

19. The process as claimed in at least one of claims 1 to 18, wherein the reducing agent is used at 10 to 200 mol%, per mole of aryl halide used.

## Revendications

1. Procédé de préparation de biaryles de formule générale (1)
R¹ₘ - Ar - Ar-R¹ₘ (1),
dans laquelle Ar signifie un reste phénylène ou naphtylène, R¹ signifie un atome de fluor ou de chlore ou bien un reste (alkyle en C₁-C₆)-SO₂-, (alkyle en C₁-C₆)-CO-, (alkyle en C₁-C₆)-O- ou bien alkyle en C₁-C₆ non ramifié ou ramifié, et m signifie le nombre de restes R¹ (positions 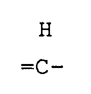 substituées sur le reste Ar), caractérisé en ce que l'on réalise une déhalogénation et une dimérisation d'un composé de formule générale (2)
R¹ₘ - Ar - X (2),
dans laquelle Ar, R¹ et m ont les significations mentionnées précédemment et X représente un atome de chlore ou de brome, en présence d'un catalyseur à base de palladium sur un matériau support, d'un agent de réduction, d'un accepteur d'halogénure d'hydrogène, d'un polyéther ou d'un mélange de polyéther et d'eau à des températures comprises entre 50 et 120°C.

2. Procédé selon la revendication 1, caractérisé en ce que R¹ est un atome de fluor dans le cas où m vaut 1.

3. Procédé selon la revendication 1, caractérisé en ce que R¹ est un atome de fluor dans le cas où m vaut 2.

4. Procédé selon la revendication 1, caractérisé en ce que, dans le cas où m vaut 2, un des R¹ sur le même reste Ar est un atome de fluor, l'autre R¹ est un groupe méthyle.

5. Procédé selon au moins l'une des revendications 1 à 4, caractérisé en ce qu'on travaille en présence d'un polyéther de formule générale
R³ - (OCH₂ - CH₂ -)ₚ OR⁴
dans laquelle R³, R⁴ représentent un reste alkyle en C₁-C₆ linéaire ou ramifié, et p représente un nombre compris entre 1 et 20.

6. Procédé selon au moins l'une des revendications 1 à 5, caractérisé en ce qu'on travaille dans un mélange de polyéthers de formule générale mentionnée dans la revendication 5.

7. Procédé selon au moins l'une des revendications 1 à 6, caractérisé en ce qu'on travaille en présence de 0,1 à 500 % en poids d'un polyéther ou d'un mélange de polyéthers, par rapport à l'halogénoaryle utilisé.

8. Procédé selon au moins l'une des revendications 1 à 6, caractérisé en ce qu'on travaille en présence de 1 à 100 % en poids d'un polyéther ou d'un mélange de polyéthers, par rapport à l'halogénoaryle utilisé.

9. Procédé selon au moins l'une des revendications 1 à 8, caractérisé en ce qu'on travaille en présence d'un composé inorganique de métal alcalin ou de métal alcalino-terreux ou bien de méthylate de sodium en tant qu'accepteur d'halogénure d'hydrogène.

10. Procédé selon au moins l'une des revendications 1 à 9, caractérisé en ce qu'on travaille en présence de 0,5 à 10 moles d'accepteur d'halogénure d'hydrogène, par rapport à une mole d'halogénoaryle utilisé.

11. Procédé selon au moins l'une des revendications 1 à 10, caractérisé en ce qu'on travaille en présence d'une solution aqueuse de 5 à 50 % en poids de l'accepteur d'halogénure d'hydrogène.

12. Procédé selon au moins l'une des revendications 1 à 10, caractérisé en ce qu'on travaille en présence d'une solution aqueuse de 15 à 40 % en poids de l'accepteur d'halogénure d'hydrogène.

13. Procédé selon au moins l'une des revendications 1 à 12, caractérisé en ce qu'on travaille en présence d'un catalyseur à base de palladium sur charbon actif ou sur carbonate de calcium.

14. Procédé selon au moins l'une des revendications 1 à 13, caractérisé en ce qu'on travaille en présence de 0,1 à 10 % en poids de catalyseur à base de palladium, par rapport au matériau support.

15. Procédé selon au moins l'une des revendications 1 à 13, caractérisé en ce qu'on travaille en présence de 0,001 à environ 50 mmoles de palladium, par rapport à une mole d'halogénoaryle utilisé.

16. Procédé selon au moins l'une des revendications 1 à 15, caractérisé en ce qu'on travaille en présence d'un catalyseur à base de palladium sur un matériau support utilisé déjà plusieurs fois dans les déshalogénations et les dimérisations.

17. Procédé selon au moins l'une des revendications 1 à 16, caractérisé en ce qu'on travaille en présence de monoalcools ou de polyalcools, de formaldéhyde, de formiate ou d'hydrazine en tant qu'agents de réduction.

18. Procédé selon au moins l'une des revendications 1 à 16, caractérisé en ce qu'on travaille en présence de méthanol, de glycérol, d'éthylèneglycol, de formaline, de paraformaldéhyde ou de formiate de sodium en tant qu'agents de réduction.

19. Procédé selon au moins l'une des revendications 1 à 18, caractérisé en ce qu'on travaille en présence de 10 à 200 % en moles de l'agent de réduction, par rapport à 1 mole d'halogénoaryle utilisé.
